(19) **Europäisches Patentamt / European Patent Office / Office européen des brevets**

(11) **EP 1 372 681 B1**

(12) **EUROPEAN PATENT SPECIFICATION**

(45) Date of publication and mention of the grant of the patent:
**01.08.2007 Bulletin 2007/31**

(51) Int Cl.:
*A61K 36/185* [(2006.01)]   *A61P 9/06* [(2006.01)]
*A61P 21/02* [(2006.01)]   *A61P 11/06* [(2006.01)]

(21) Application number: **01929981.7**

(22) Date of filing: **29.03.2001**

(86) International application number:
**PCT/IN2001/000059**

(87) International publication number:
**WO 2002/078725 (10.10.2002 Gazette 2002/41)**

(54) **BIOLOGICALLY ACTIVE CHLOROFORM FRACTION OF AN EXTRACT OBTAINED FROM A MANGROVE PLANT SALVADORA PERSICA L**

BIOLOGISCH WIRKSAME CHLOROFORM FRAKTION EINES EXTRAKTES ERHALTEN AUS DER MANGROVENPFLANZE SALVADORA PERSICA L

FRACTION DE CHLOROFORME BIOACTIVE D'UN EXTRAIT OBTENU D'UNE PLANTE DE MANGROVE SALVADORA PERSICA L

(84) Designated Contracting States:
**DE FR GB**

(43) Date of publication of application:
**02.01.2004 Bulletin 2004/01**

(73) Proprietor: **COUNCIL OF SCIENTIFIC AND INDUSTRIAL RESEARCH**
**New Delhi 110 001 (IN)**

(72) Inventors:
 • **GOSWAMI, Usha,**
   **National Institute of Oceanography**
   **Goa 403 004 (IN)**
 • **FERNANDES, N.,**
   **National Institute of Oceanography**
   **New Delhi 403 004 (IN)**

(74) Representative: **Portal, Gérard et al**
**Cabinet Beau de Loménie**
**158, rue de l'Université**
**75340 Paris Cédex 07 (FR)**

(56) References cited:
 • **DATABASE BIOSIS [Online] BIOSCIENCES INFORMATION SERVICE, PHILADELPHIA, PA, US; August 2000 (2000-08) KAMIL M ET AL: "Isolation and identification of a flavonol glycoside using high speed counter current chromatographic technique from the leaves of Salvadora persica." Database accession no. PREV200000489475 XP002183227 & PAKISTAN JOURNAL OF SCIENTIFIC AND INDUSTRIAL RESEARCH, vol. 43, no. 4, August 2000 (2000-08), pages 255-257, ISSN: 0030-9885**
 • **L. M. KAMIL ET AL.: "PHARMACOGNISTICAL AND PHYTOCHEMICAL STUDIES ON SALVADORA PERSICA L." JOURNAL OF PHARMACY AND PHARMACOLOGY, vol. 51, no. SUPPL., September 1999 (1999-09), page 227 XP001031401 LONDON, GB ISSN: 0022-3573**

## Description

### Technical Field

The invention relates to biologically active chloroform fraction of extracts obtained from the plant *Salvadora persica Linneaus* 1753. The invention also provides a process for obtaining the crude extract and obtaining the chloroform fraction. Further, the invention provides pharmaceutical compositions, exhibiting biological activity.

### Background Art

The associated mangrove plant, *Salvadora persica Linneaus,* 1753, belongs to the order Salvadoracea and are shrubs or small trees with white flowers frequent in degraded mangrove swamps and saline banks all over the west coast of India. Large numbers of marine plants have been examined for bioactive substances. Nazarine, F; Anita F; Rataboli, P.V.; Diniz D'Souza, R. S and Dhume; V.G., 1998 in Indian Journal of marine Sciences, 27: 499-501 have reported promising pharmacological activities in marine organisms from Indian waters.

There are several patents available from all over the world related to processes and compounds from nature for various purposes. Kwak; Wie-jong; Han; Chang-kyun; kum; Hwan-su; An; Jae-suk; Kum; Taek-soo, patented process of extracting and purifying biologically effective ingredients from combined medicinal plant and their extract composition (United States Patent No.5,910,307 published on June 8, 1999). D'Amelio; Frank S; Mirhom; Youssef W. disclosed therapeutic composition and method for treating skin using extract from *Centipeda cunninghami* plant in US Patent No. 5,804,206 published on September 8, 1998. Zimmerman; Richard C.; Alber; Randall S.; Todd; James S.; Crews; philip, isolated compound from the methanolic extract of the eelgrass *Zostrea marina* having significant antifouling aquatic properties (US Patent No.5,607,741 published on March 4, 1997).

Betulinic acid which is prepared from the compound Betulin has many pharmaceutical potentials. Pezzuto John M; Kim; Darrick S.H.L. disclosed methods of manufacturing betulinic acid from betulin (US Patent No.5,804,575 published on 8 September, 1998). The betulinic acid is intensively investigated as a potential therapeutic agent for a variety of diseases. Pezzuto; John, M; Das Gupta, Tapas K; Schmidt; Mary Lou; Kuzmanoff; Konrad Marc; Ling Indeck; Lydia and Kim; Darrick, S.H.L. (US Patent No.5,962,527 dated October 5, 1999). Pisha E, Chai H, Lee IS, Chagwedera TE, Framsworth NR, Cordell GA, Beecher CW, Fong H H, Kinghorn AD, Brown DM, in Nature medicine, 1 pages 1046-1051 (1995) discloses that betulinic acid has an unexpected selective antitumour activity against human melanoma e.g. MEL-1, MEL-2 and MEL-4. In addition Fujioka T, Kashiwada Y, Kilkuskie RE, Cosentino LM, Ballas LM, Jiang JB, Janzen WP, Chen IS. Lee KH. J.Nat.Prod., 57 (2) pages 243-247 (1994) discloses that betulinic acid has anti-HIV activity in H9 lymphocytic cells. These inventors mentioned that the researches directed t betulinic acid as therapeutic agent are hindered because the betulinic acid is available in very limited quantities and at a very high cost. Ramadoss, Sunder, Jaggi, Manu; Siddiqui; Mohammad Jamshed Ahmed in US Patent No.6,048,847 published on April 11, 2000 describes uses of betulinic acid and its derivatives for inhibiting cancer growth and a method of monitoring this. Kang; Raphael K.L; Zyzak; Li Li; nakatsu; Tetsuo published flavoured product additives in US Patent No.5,948,460, dated 7 September, 1999 in which Ursolic acid was one of the compound amongst a group of three compounds which was added to a flavoured product to reduce aftertaste in the product and enhance its sweetness like in a diet drink. It was also used as a constituent in a preparation for inhibition of skin Tumorigenesis.

Herman. S (US Patent No-5,190,979 published on 2 March, 1993) disclosed lupeol also as a compound which can make pharmacologically active terpene ozonides which have medicinal value.

### Objects of the invention

The main object of the present- invention is to provide a process for obtaining the chloroform fraction from the crude extract from stem, leaves, and flowers of *Salvadora persica,* a commonly available shrub in mangrove swamps and screening to study its bioactivity.

Another object disclosed by the invention is to isolate naturally occurring compounds from the plant *Salvadora persica* and identify their molecular weights, molecular formulae, melting points and their structural formulae, which will be helpful in chemical synthesis of these compounds.

Yet another object disclosed by the invention relates to the pharmacological screening of the chloroform fraction of the crude extract, its fractions and purified compounds to check that the activities shown by the crude extract and chloroform fraction are maintained throughout.

Another object of the invention is to provide pharmaceutical compositions containing chloroform fraction of the extract from the plant *Salvadora persica* and exhibiting biological activity.

Still another object of the invention is the use of an effective amount of a chloroform fraction of the extract obtained from the plant *Salvadora persica* in the manufacture of a medicament for treating arrhythmias and/or spasmodic conditions and/or bronchial asthma in a human or animal subject.

## Summary of the invention

The present invention seeks to overcome the drawbacks inherent in the prior art by providing the highly efficient and selective means for processing of active crude extract its fractionation, isolation and purification of the active compounds. Further, the invention provides pharmaceutical compositions containing the extract obtained from the plant *Salvadora persica.*

## Detailed description of the invention

The present invention discloses for the first time the methods of isolation, purification and pharmacological screening of all these above said commercially important compounds from a commonly available plant from mangrove swamps of the west coast of India. The said plant identified as *Salvadora persica,* is an associated mangrove plant. It is a shrub and the twigs with leaves of flowers can be hand picked. Even the crude extract and fractions exhibit therapeutic value. The important point is that the biological activity shown by the extract is maintained in the purified compounds such as Methyl palmitate and Betulin.

This disclosure points towards future potential clinical uses of the extract and fractions for treatment of diseases such as smooth muscle relaxant, bronchial asthma, renal colics and prevention of premature delivery. It further relates to the use of Methyl palmitate and Betulin in motion sickness, abdominal cramps.

The invention provides methods whereby biologically active crude extract of an associated mangrove plant identified as *Salvadora persica Linneaus* 1753 is prepared. *Salvadora persica Linneaus* (Salvadoraceae) are shrubs or small trees with white flowers frequent in degraded mangrove swamps and saline banks all over the west coast of India; west Asia. The process disclosed in the invention further relates to the extraction, fractionation and purification of active constituent metabolites of the said plant. The invention is also concerned with the spectral identification of the compounds such as β-amyrin (non-steroidalpolycyclic triterpene), Betulin, Ursolic acid (triterpenic acid), Methyl palmitate (Aliphatic Ester) and Lupeol (non-steroidalpolycylic triterpene). The invention also deals with molecular formulae, molecular weights, melting points and structural formulae of the said compounds. The invention provides a highly efficient and selective means for processing of active crude extract obtained from *Salvadora persica,* its fractionation, isolation and purification. As used herein, the terms fractionation means separating the crude extract. The term isolation and fractionation means separating the fraction into pure compounds.

The invention further discloses to methods of screening pharmacological activities of the said compounds in mammalian tissues. The applicant has found that the crude extract obtained from the plant *Salvadora persica* can be separated into two fractions, i.e., chloroform and aqueous fractions. The chloroform fraction exhibits antispasmodic, anti-cholinergic and anti-arrhythmic activities which is described in detail in the present invention.

The invention has a different approach for pharmacological screening of anti-arrhythmic activity on atrial tissues. The left atrium was used for electric stimulations rather than the right atrium which has the pacemaker. This method was found advantageous for longer survival of the atrium during experiments.

Accordingly, the invention discloses a process of extracting and purifying biologically useful molecules from an associated mangrove plant which comprises the steps of:

> i) collecting and processing the plant plants of *Salvadora persica,*
> ii) preparing a crude extract from the plant parts of *Salvadora persica*,
> iii) testing the crude extract using methods of pharmacology,
> iv) fractionating the crude extract,
> v) testing the fractions using methods of pharmacology,
> vi) isolating the pure compounds by column chromatography,
> vii) testing the pure compounds by using methods of pharmacology, and
> viii) identifying the compounds by spectroscopy.

According to another embodiment of the invention, the chloroform fraction of the mangrove plant *Salvadora persica* showed arrhythmic, anti-spasmodic, anti-cholinergic activity, as the parent crude extract.

The invention also provides the identification of the molecules from the spectral data. The molecular formulae of the five compounds is provided from the spectral data. The invention provides molecular weights of the molecules from EIMS. The structural formulae of the compounds is also provided from the spectral data.

Thus, a crude extract was obtained from the plant *Salvadora persica.* The crude extract was tested for its bioactivity and if found promising in terms of its pharmacological activity, it was fractionated using solvents with increasing polarity to obtain fractions such as petroleum ether, chloroform, butanol and aqueous. Each of these were also tested for their pharmacological activity.

The five compounds purified from the extract of the plant were β-amyrin (non - steroidalpolycyclic triterpene ), Betulin, Ursolic acid (triterpenic acid), Methyl palmitate (Aliphatic Ester ) and Lupeol (non-steroidalpolycyclic triterpene).

β-amyrin was found to be a non-steroidalpolycyclic triterpene with the following details:

| | |
|---|---|
| Molecular formula | : $C_{30} H_{50} O$ |
| Molecular weight | : 426 |
| Melting point | : 160.degree C |

Another molecule found was betulin having:

| | |
|---|---|
| Molecular formula | : $C_{30} H_{50} O_2$ |
| Molecular weight | : 442 |
| Melting point | : 255 degree.C |

Ursolic acid (triterpenic acid) molecule was also found in the extract. It had:

| | |
|---|---|
| Molecular formula | : $C_{30} H_{48} O_3$ |
| Molecular weight | : 456 |
| Melting point | : 292 degree.C. |

Methyl palmitate (Aliphatic Ester ) found in the extract had:

| | |
|---|---|
| Molecular formula | : $C_{16} H_{32} O_2$ |
| Molecular weight | : 256 |
| Melting point | : 30 degree.C |

Lupeol (non-steroidalpolycyclic triterpene) found in the extract had:

| | |
|---|---|
| Molecular formula | : $C_{30} H_{50} O$ |
| Molecular weight | : 426 |
| Melting point | : 215 degree. C |

One of the compounds Betulin which can be used in manufacturing of betulinic acid (US Patent No.5,804,575 published on 8 September, 1998. Betulinic acid is intensively investigated as a potential therapeutic agent for a variety of diseases.

Ursolic acid is added to a flavoured product to reduce after taste in the product and enhance its sweetness for example in a diet drink. It was also used as a constituent in a preparation for inhibition of skin Tumorigenesis.

Extracts of some plants show vasoconstrictor and analgesic properties and also contain triterpenoid beta.-amyrin. These compositions for inhibiting the formation of unwanted skin pigmentation combine high tyrosinase blocking capabilities with stability in cosmetic preparations, absence of significant cytotoxic effects and synergy of action (US patent 5,773,014, 5,679.393). Beta-amyrin and lupeol are used as components for dimethylsterols in medical formulations (US Patent 4,808,574).

Methyl palmitate is compound used in making alcohols as mentioned in US patent No. 6,049,013 published on April. 2000. Lupeol can be used as a component for several remedial medicines, insect repellants, distilleries anti tumour and chemical industries (US Patent No. 4,808,574; 5,962,527; 5.908,628).

Thus, the extract of *Salvadora persica* contained several compounds as listed above. The chloroform fraction of the extract of the said plant *Salvadora persica,* was then tested for its biological activity on guinea pigs. The Applicants, to their surprise found that the extract exhibited excellent anti-cholinergic, anti-arrhythmic and anti-spasmodic activity.

Accordingly, the invention provides compositions containing the chloroform fraction of the extract obtained from *Salvadora persica,* optionally with conventional additives for inhibition of treatment of anti-cholinergic conditions such as asthma. The composition may contain about 10 μgm of the extract. Thus, the chloroform fraction of the extract obtained from the plant *Salvadora persica* is a potential anti-cholinergic agent. The compositions may be formulated in different physical forms, as may be required. The extract may be used as such or with conventional additives, physiologically acceptable carriers, preservatives buffers, etc. as required.

Additionally, the invention discloses the use, for the manufacture of a medicament for treating anti-cholinergic conditions such as bronchial asthma, of therapeutically effective amount of extract obtained from *Salvadora persica.* The extract may be administered at a dosage level in the range of 50 $\mu$g/ml to 250$\mu$g/ml, in case of normal adults. The exact dosage will vary depending on the patient to be treated and will depend on factors such as requirements of the patient, severity of the condition being treated and the activity of the extract. The determination of optimum dosages for a particular patient is well-known to those skilled in the art.

Further, the applicant found that the chloroform fraction of the extract obtained from the plant *Salvadora persica* exhibits anti-spasmodic activity. Accordingly, the invention provides compositions containing the chloroform fraction of the extract obtained from *Salvadora persica,* optionally with conventional additives for inhibition of treatment of spasmodic condition such as muscular spasms. The composition may contain about 10 $\mu$gm of the extract. Thus, the chloroform fraction of the extract obtained from the plant *Salvadora persica* is a potential anti-spasmodic agent. The composition may be formulated in different physical forms, as may be required. The extract may be used as such or with conventional additives, physiologically acceptable carriers, preservatives, buffers, etc. as required.

Additionally, the invention discloses the use, for the manufacture of a medicament for treating spasmodic conditions such as bronchial asthma, of therapeutically effective amount of extract obtained from *Salvadora persica.* The extract may be administered at a dosage level in the range of 50 $\mu$g/ml to 250$\mu$g/ml, in case of normal adults. The exact dosage will vary depending on the patient to be treated and will depend on factors such as requirements of the patient, severity of the condition being treated and the activity of the extract. The determination of optimum dosages for a particular patient is well-known to those skilled in the art.

Furthermore, the applicant found that the chloroform fraction of the extract obtained from the plant *Salvadora persica* exhibits anti-arrhythmic activity. Accordingly, the invention provides compositions containing the chloroform fraction of the extract obtained from *Salvadora persica,* optionally with conventional additives for inhibition of treatment of arrhythmias of the heart. The composition may contain about 10 $\mu$gm of the extract. Thus, the chloroform fraction of the extract obtained from the plant *Salvadora persica* is a potential anti-arrhythmic agent. The compositions may be formulated in different physical forms, as may be required. The extract may be used as such or with conventional additives, physiologically acceptable carriers, preservatives, buffers, etc. as required.

Additionally, the invention discloses the use, for the manufacture of a medicament for treating arrhythmias of the heart, of therapeutically effective amount of extract obtained from *Salvadora persica.* The extract may be administered at a dosage level in the range of 3 $\mu$g/ml to 10 $\mu$g/ml, in case of normal adults. The extract is administered to the subject for a period of 10 minutes. The exact dosage will vary depending on the patient to be treated and will depend on factors such as requirements of the patient, severity of the condition being treated and the activity of the extract. The determination of optimum dosages for a particular patient is well-known to those skilled in the art.

## Detailed description of the accompanying drawings

| | |
|---|---|
| **Fig 1(a)** | shows the mangrove plant and |
| **Fig 1(b)** | shows the twig of the associated mangrove plant used |
| **Fig 2** | shows the different fractions obtained from the extract of the plant *Salvadora persica.* |
| **Fig.3** | shows the structural formula of $\beta$-amyrin (non - steroidalpolycyclic triterpene ) |
| **Fig.4** | shows the structural formula of Betulin, |
| **Fig 5** | shows the structural formula of Ursolic acid (triterpenic acid), |
| **Fig.6** | shows the structural formula of Methyl palmitate (Aliphatic Ester ) |
| **Fig.7** | shows the structural formula of Lupeol (non-steroidalpolycyclic triterpene). |
| **Fig.8** | shows the fractionation chart of extract of *Salvadora persica* |

The invention is described in detail and illustrated by the following examples which should not be construed as limitations on the inventive concept embodied herein.

**Example 1 : This example provide information about the Chemicals, Reagents, Apparatus used and their sources.**

| Name of reagent/chemicals | Company |
|---|---|
| Aqueous methanol | Sisco Research Laboratories Pvt Ltd. |
| Petroleum ether | Ranbaxy Fine Chemicals Ltd. |
| Chloroform | Sisco Research Laboratories Pvt Ltd. |
| Butanol | Sisco Research Laboratories Pvt Ltd. |
| Ethyl acetate | Sisco Research Laboratories Pvt Ltd. |
| Histamine acid phosphate | Blenkinsop & Co. Ltd |
| Acetylcholine chloride | Hopkin & Williams Ltd. |
| 5-Hydroxytryptamine creatine sulphate | Sigma Chemicals |
| Barium chloride | Apex Chemicals |
| Nicotine sulphate | BDH chemicals |
| Oxytocin | Parke Davis India Ltd. |
| Prostodin-PGF 2 alpha | Astra IDL Ltd. |

**APPARATUS:**

**1.** Physiograph .
Company: Biodevices,
Ambala, India.
**2.** Force Transducer
Model No. T-305
Co.: GRASS,USA
**3.** Stimulator
Model SS44
Co. Biodevices, Ambala
**4.** Polygraph
Model 7
Co. GRASS, USA.
**5.** Force Transducer
Model No. FT-03
Co.: GRASS,USA
**6.** Organ Bath
Ambala,
India

**Example 2**

Collection of the mangrove plant *Salvadora persica* L from the coast of Goa, a state in India was along Ribandar, near the mouth of the Mandovi estuary, upstream. This species is ubiquitous to the coastal areas of Goa and was collected manually from the intertidal banks.

**Example 3**

Processing of the collected mangroves were washed first with seawater followed by tap water. The undesired materials were sifted out while washing with tap water to get rid of the salts. The leaves, stems, and flowers of the associated mangrove plant were air dried. After drying, the plant material was cut into small pieces and immersed in the solvent 90% aqueous methanol for a week for extraction. Care was taken to ensure that these were properly soaked/ dipped in the solvent and checked for putrefaction.

**Example 4**

Extraction and preparation of crude extract was carried out by cold percolation method at room temperature and by solvent evaporation at a water bath (temperature 50°C) under reduced pressure. This helps in protection of any heat labile metabolite present in it. Re-extraction was done twice until the extract was concentrated under vacuum to obtain the crude extract.

### Example 5

**Fractionation of the crude extract**

The crude extract was partitioned into petroleum ether, chloroform, n-butanol and aqueous fractions using a separating funnel. Petroleum ether was added to the extract in the separating funnel and separated out. Next, chloroform was added to the residue, mixed well and the lower layer separated. To the residue butanol was added and the top layer represented the butanol fraction and the lower layer was the aqueous fraction. Extraction of each fraction was done thrice and whenever there was emulsion, sodium chloride was added for breaking the emulsion. Sodium sulphate was added to chloroform and butanol fractions to remove traces of water before concentration. All the fractions were concentrated in the same manner as the crude extract. These fractions were tested for the same pharmacological activity as the parent crude extract. Column chromatography for isolation of pure compound was done by repeated column chromatography and thin layer chromatography of the eluents. The TLC revealed compounds such as Beta-amyrin, betulin, ursolic acid and lupeol.

### Example 6

To obtain the compounds Beta amyrin and betulin, separation by thin layer chromatography is carried out on 0.25mm thick silica gel plates (Qualigen). The eluent is an 90:10 (v/v) Petroleum ether/ethyl acetate mixture and the spots are developed by spraying with 5% $H_2SO_4$ solution and fixation by heating at 110.degree. C.

For compounds Ursolic acid and Methyl palmitate separation by thin layer chromatography was carried out on 0.25 mm thick silica gel plates (Qualigen). The eluent is an 85:15 (v/v) Petroleum ether/ethyl acetate mixture and the spots are developed by spraying with a 5% $H_2SO_4$ solution and fixation by heating at 110°C.

For the compound lupeol the separations by thin layer chromatography are carried out on 0.25 mm thick silica gel plates (Qualigen). The eluent is an 75:25 (v/v) Petroleum ether/ethyl acetate mixture and the spots are developed by spraying with a 5% $H_2SO_4$ solution and fixation by heating at 110°C.

In the present invention, the active chloroform fraction of the mangrove plant S. persica , was column chromatographed over silica gel for the isolation of the active constituent. Elutes from the column with the same TLC profile were mixed and subjected to pharmacological testing. The active sub-fractions were further chromatographed till active pure compounds were obtained (Flow chart I). Spots on thin layer chromatography (TLC) were visualised by using iodine vapours and spraying with methanolic sulphuric acid.

TLC was done on glass plates (20 X 20 cms) coated with a 0.25mm layer of TLC grade silica gel (Qualigens) activated at 110° C for 1 hour before use. The active aqueous fraction was passed through XAD-column and eluents were treated, as mentioned above, for the isolation and purification of active constituent metabolites.

The five compounds were identified on the basis of spectral data obtained at the Regional Sophisticated Instrumentation Center ( RSIC) India by the following spectra :

$^1$HNMR for determining the proton environment of the molecule carried out on Bruker DPX-200 MHz.
**Apparatus:** Bruker Spectrometer
Model: DPX
Co. Bruker
$^{13}$ CNMR for carbon atoms Bruker DPV 300 MHz.

The compounds were identified from a comparison of their spectral data with those of similar compounds reported in literature .

Mass spectra (EIMS) electron impact Mass spectrometry for determining the molecular weights along with its fragmentation pattern were carried out on Mass spectrometer (E1/CIMS) Model D.300 JEOL,

**Apparatus:** Mass Spectrometer (EIMS)
Model: D-300
Co. JEOL, Japan

### Example 7

Pharmacological testing of pure compounds

Standarddrugs used were the following:

Histamine acid phosphate (Blenkinsop & Co. Ltd) on ileum.
Acetylcholine chloride (Hopkin & Williams Ltd) on ileum.
5-Hydroxytryptamine creatinine sulphate (sigma Chemicals Co.) on gastrointestinal tract. Barium chloride (Apex Chemicals) on smooth muscle contraction.
Nicotine sulphate (BDH Chemicals) on intestine as ganglion stimulant.

All other reagents used were of analytical grade.
Tyrode was used on guinea-pig ileum and Ringer-Locke physiological solution was used on guinea-pig atria.
All other reagents used were of analytical grade.
Physiological solutions used and various parameters:

All physiological solutions were prepared fresh at the time of the experiment.
pH: The pH of the various physiological salt solutions varied between 7.3 & 7.4. At lower pH the tonus of the preparation tends to decrease and is therefore liable to alter the effect of drugs.
Temperature: In order to get consistent effects it was important to maintain the temperature of the bath solution at a specified level, because if the temperature is decreased below 37°C, the tonus of the intestine is increased, the contractions become smaller and the contraction and relaxation times increased.
Air : Air or oxygen is needed for proper functioning of the tissues. Besides, providing oxygen to the tissues, the stream of gas bubbles also stirred the bath solution thereby facilitating diffusion of drugs added to the bath.

The solution in the bath was changed frequently because prolonged aeration tends to alter the pH.

### In vitro experiments

Female, virgin, guinea pigs weighing around 300 to 350 g, housed under uniform husbandry conditions (temperature 25 $\pm$ 1°C) were used. The animals were starved 24 hours prior to the experiment, only water was provided *ad libitum.*
The isolated guinea pig ileum was used to study the anti-spasmodic, anti-cholinergic and anti-arrhythmic activity.

## A. ANTI-CHOLINERGIC ACTIVITY

The active fractions were isolated for anti-cholinergic activity, the five compounds isolated were tested only on isolated guinea-pig ileum. The longitudinal ileal muscle from a freshly killed guinea-pig was suspended in an organ bath of 10 ml capacity, filled with Tyrode solution and aerated with air. Tyrode solution comprised of:

**Tyrode solution**

| | |
|---|---|
| Glucose | 1.0 g |
| Sodium chloride | 8.0 g |
| Sodium bicarbonate | 1.0 g |
| Potassium chloride | 0.2 g |
| Calcium chloride | 0.2 g |
| Magnesium chloride | 0.1 g |
| Sodium hydrogen phosphate | 0.05 g |

All were dissolved in 1000 ml distilled water
Two to four doses of the standard drug acetylcholine were added to the bath to obtained uniform amplitude with a contact period of 30 seconds, the contractions of which were recorded on a polygraph. The five compounds isolated were tested in doses of 196 and 392 X $10^{-6}$ moles per ml of bath concentration. The effect of the compounds against acetylcholine - induced contraction was seen and the percentage reduction of contraction measured.

## B. ANTI-SPASMODIC ACTIVITY

The active fractions were isolated for anti-spasmodic activity, the five compounds isolated were tested only on isolated guinea-pig ileum. The longitudinal ileal muscle from a freshly killed guinea-pig was suspended in an organ bath of 10 ml capacity, filled with Tyrode solution and aerated with air.

For pharmacological testing on guinea pig ileum, the guinea-pig was sacrificed by stunning with a sharp blow on its head. The abdomen was quickly cut open. Towards the lower end of the abdomen was the greenish sac-like caecum. The small intestine was marked by a localized thickening in the wall - a Peyer's patch of lymphoid tissue. The lowermost 10 cm of ileum nearest to the ileocaecal junction was discarded. From there, about 10 cm of ileal tissue was cut off and freed of mesentery and placed in a petridish containing warm Tyrode solution. The lumen of the ileum was gently rinsed out using a hypodermic syringe filled with Tyrode solution to prevent accumulation of mucus in the lumen. The ileum was cut into small segments of about 3-4 cms in length in the fully relaxed state. The lower end was sutured to a tissue holder, by making a loop first to avoid direct contact with the tube. The tissue was positioned in an organ bath of capacity 10 ml containing Tyrode solution aerated with air at 37°C. The thread of the upper end of the ileum was fixed to the lever of a force transducer (FT 03) which measures muscle contractions isometrically and connected to a Grass Polygraph (Model 7). The ileal tissue was kept to stabilize in Tyrode solution for 30 minutes and the fluid in the organ bath was renewed every 10 mins (as pH changes).

Two to four doses of the standard drug spasmogens (acetylcholine, histamine, 5-hydroxytryptamine, barium chloride and nicotine) were added to the bath to obtain uniform amplitude with a contact period of 60 seconds, the contractions of which were recorded on a polygraph. The chloroform extract was added one minute before addition of the spasmogens. The effect of the compounds against acetylcholine - induced contraction was seen and the percentage reduction of contraction measured .

**TABLE SHOWING RELATIVE IN VITRO ANTISPASMODIC EFFECT OF *SALVADORA PERSICA L.* CRUDE EXTRACT ON GUINEA-PIG ILEUM (Mean of 0 readings)**

| EXTRACT | TISSUE | SPASMOGENS USED | STANDARD INDUCED CONTRACTION (mm) | DOSE 50mg / ml (mm) | INHIBITION % DECREASE | DOSE 250 mg/ml | INHIBITION % DECREASE |
|---|---|---|---|---|---|---|---|
| CRUDE EXTRACT | GUINEA PIGILEUM | ACETYLCHOLINE | 20 | 15 | 25 | 8 | 60 |
| | | HISTAMINE | 22 | 18 | 18.17 | 15 | 32 |
| | | NICOTINE | 16 | 16 | 0 | 10 | 37.5 |
| | | BARIUM | 18 | 16 | 11 | 12 | 33 |
| | | CHLORIDE | | | | | |

## C. ANTI-ARRHYTHMIC ACTIVITY

In Vitro experiments were performed on isolated guinea pig atria.

The thorax of the stunned guinae pig was quickly opened by cutting the sternocostal junctions. The heart was seen behind the sternum, beating in its pericardial covering. It was nicked off and placed in a petridish containing Ringer-Locke solution aerated with pure oxygen. It was gently squeezed to remove blood from the cavities of the atria and the ventricles and to prevent clotting of blood inside the coronary arteries. All other tissues were cut away until nothing was left except the auricles which appeared as a pair of rapidly beating pale pink leafy structures. Threads were tied one to the tip of each auricle. The right atrium having the pacemaker was tied to the glass oxygen tube and mounted in an organ bath of capacity 40 ml containing Ringer- Locke solution at 34°C and oxygenated . The thread at the other end of the left auricle was fixed to the transducer (Force transducer T-305) which was connected to a Biodevices Physiograph. The composition of Ringer-Locke solution was :

| | |
|---|---|
| Glucose | 0.5g |
| Sodium chloride | 9.0g |
| Sodium bicarbonate | 0.5g |
| Potassium chloride | 0.42g |
| Calcium chloride | 0.24g |

The sensitivity on the Physiograph was adjusted according to the heart beat. The auricles were fixed in a pair of stimulating electrodes, kept vertically immersed inside the bath. The auricles were allowed to equilibrate for a period of 30 minutes. The electrode was connected to a stimulator (Medicare Research Stimulator SS44) at a duration of 5x1 misec and 5x0.1 misec delay kept constant. Initially the voltage and frequency were minimum. Then the voltage was gradually increased till there was a change in amplitude. Then the frequency was increased/adjusted to get arrhythmias. Doses of extract/fraction were given as concentrations of the salt per ml of bath solution and allowed to remain in contact with the auricles for 10 minutes. After every alternate minute, the physiograph was run to see if there was any change in heart rate. After 10 minutes the auricles were stimulated twice for arrhythmias. The anti-arrhythmic effect of each dose i.e. 3 and 10 $\mu$g/ml was calculated. The formula used for evaluating the arrythmic effect of crude extract of *Salvadora persica* on guinea pig atrium is:

$$\% \text{ inhibition} = \frac{(X) - (Y) \times 100}{(X)}$$

wherein

X= Maximum frequency before the extract

Y= Maximum frequency after the extract

**The relative *in vitro* effect of *Salvadora persica* on guinea pig atrium is shown in the Table hereinbelow:**

| EXTRACT | TISSUE | FREQUENCY BEFORE DRUG | FREQUENCY AFTER DRUG | PERCENTAGE CHANGE |
|---|---|---|---|---|
| Crude Extract | Atrium | 96 beats/sec. | 96 beats/sec. | 0 |

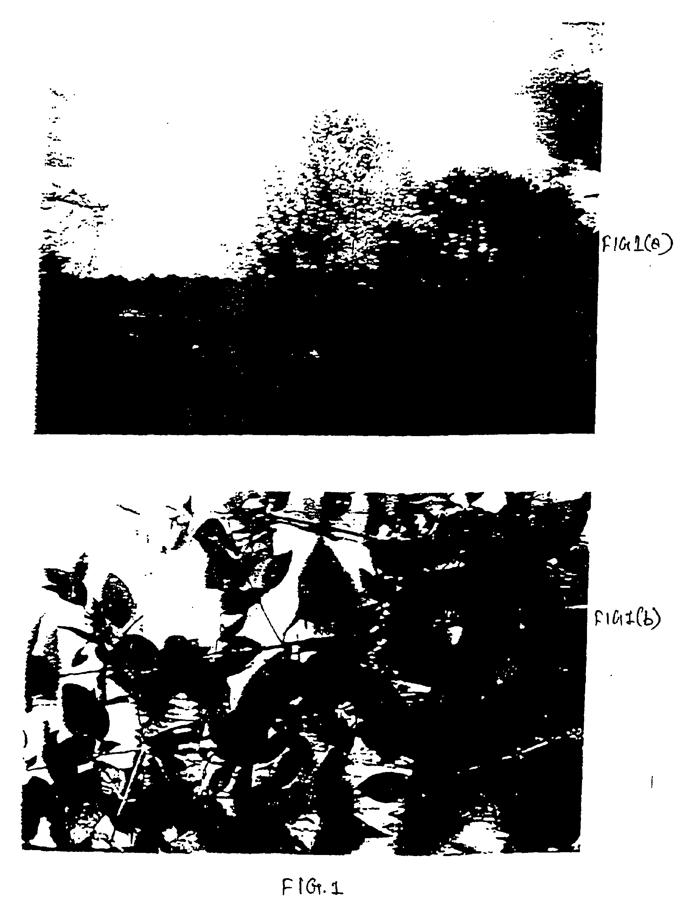

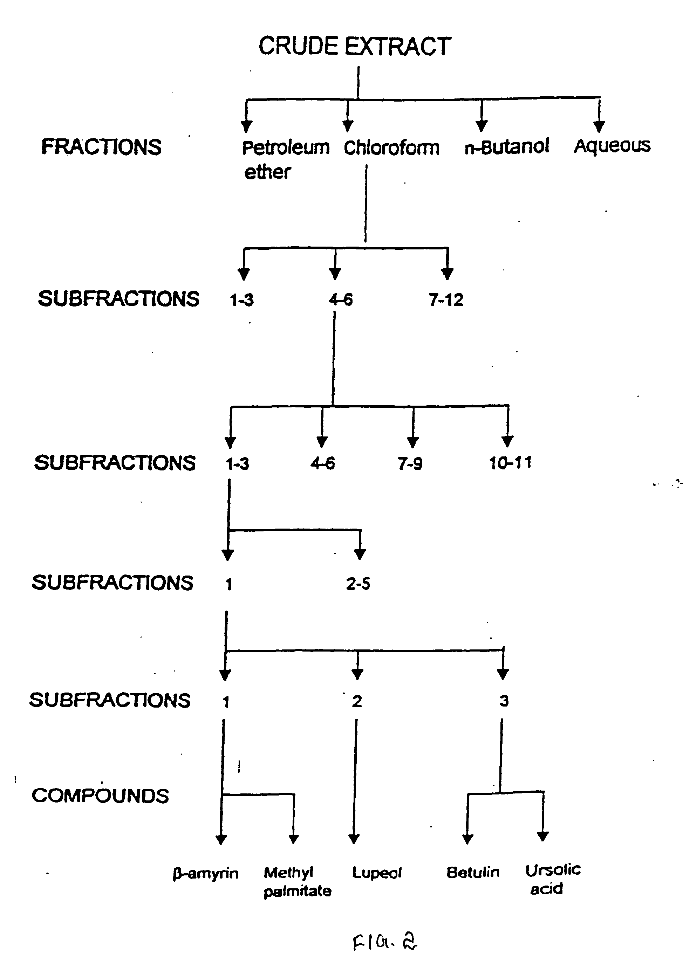

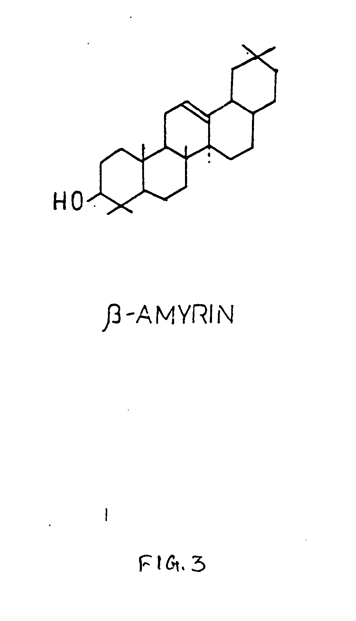

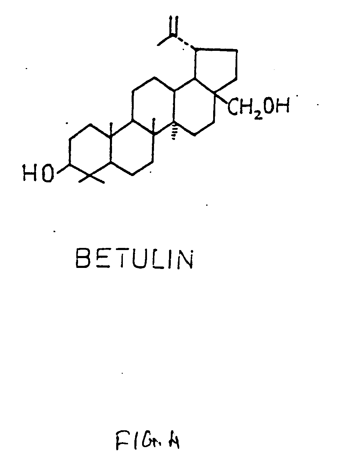

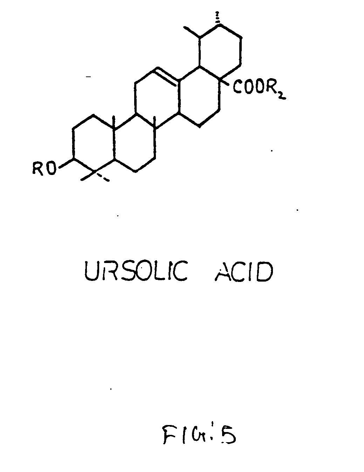

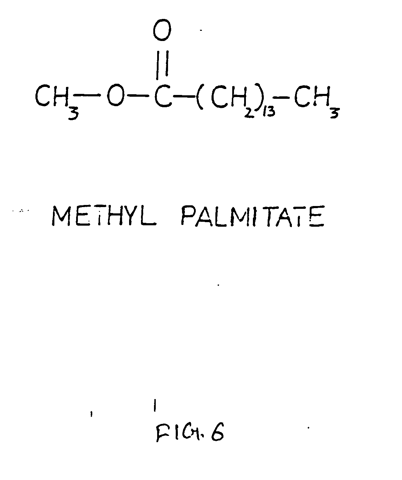

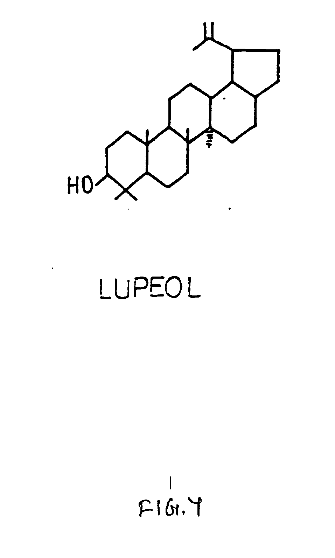

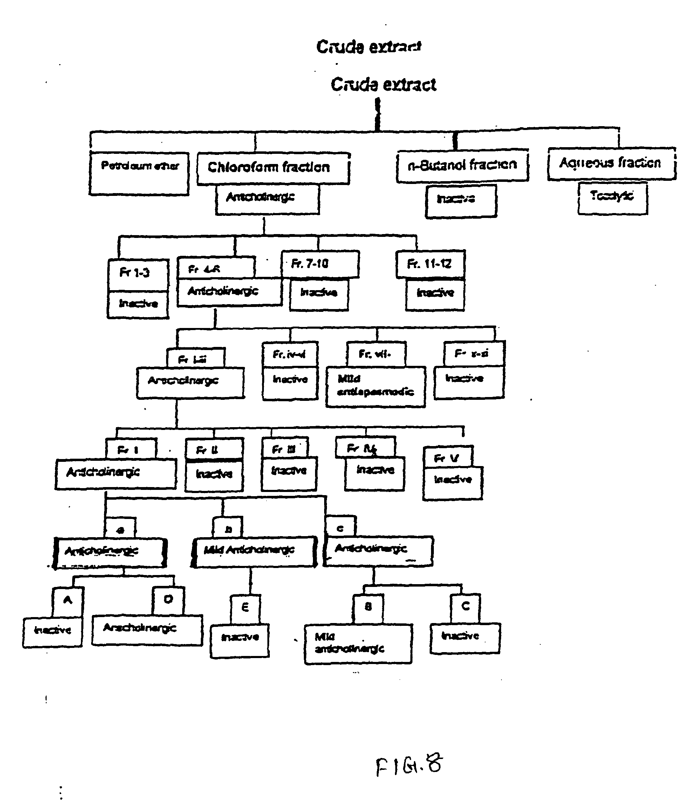

## Claims

1.    A process for the extraction, fractionation, purification, and identification of biologically active metabolites from biologically active extract of the plant *Salvadora persica,* comprising steps of :

a, collecting plant parts of *Salvadora persica,*
b. air drying the plant parts,
c. immersing the plant parts in about 90 % aqueous methanol for about one week at room temperature of about 28 $\pm$ 2°C,
d. filtering the methanolic extract,
e. evaporating the methanolic extract under reduced pressure at temperature of about 50°C, to a minimum quantity to obtain a crude extract,
f. fractionating the crude extract using solvents of increasing polarity,
g. identifying the biological active fraction, and
h. isolating biologically active compounds from the fraction of step (g) by column chromatography.

2.    A process as claimed in claim 1, wherein the plant parts of *Salvadora persica* are selected from leaves, stems and flowers.

3.    A process as claimed in claim 1, wherein solvents of increasing polarity are petroleum ether, chloroform, butanol and aqueous fraction.

4.    A process as claimed in claim 1, wherein the chloroform fraction exhibits anti-cholinergic activity, anti- spasmodic and anti-arrhythmic activity.

5.    A process as claimed in claim 1, wherein the chloroform fraction is passed through a silica gel column and then eluted using petroleum ether -ethyl acetate- chloroform-methanol gradient system.

6.    A process as claimed in claim 1, wherein the thin layer chromatography (TLC) was carried out on silica gel.

7.    A process as claimed in claim 1, wherein the TLC was visualized either by exposing plates to iodine vapors or by spraying with methanolic sulphuric acid.

8. A process as claimed in claim 1, wherein the fractions which showed anti-cholinergic activity were pooled and passed through the silica gel column and eluted with same set of solvents.

9. A Pharmaceutical composition exhibiting anti-cholinergic, anti-spasmodic, and anti-arrhythmic activity, said composition comprising the chloroform fraction of the extract obtainable from the plant *Salvadora persica,* optionally with conventional additives.

10. A composition as claimed in claim 9 wherein the additives are selected from therapeutically acceptable additives.

11. A composition as claimed in claim 9 wherein the extract contains compounds selected from β-amyrin, betulin, ursolic acid, methyl palmitate and lupeol.

12. A composition as claimed in claim 9 wherein the amount extract in the composition is 10μg.

13. A composition as claimed in claim 9 wherein the dosage of the extract is 3μg/ml to 10 μg/ml.

14. Use of a therapeutically effective amount of a chloroform fraction obtainable from the plant *Salvadora persica,* optionally with conventional additives, in the manufacture of a medicament for treating arrhythmias in a subject

15. The use of claim 14 wherein the medicament is formulated for the administration of 3 to 10 μg/ml of said chloroform fraction for a period of 10 minutes to a subject.

16. The use of claim 14 or 15 wherein the subject is a human or animal subject.

17. Use of a therapeutically effective amount of a chloroform fraction of the crude extract of the plant *Salvadora persica,* optionally with conventional additives, in the manufacture of a medicament for treating spasmodic conditions such as muscular spasms in a subject.

18. The use of claim 17 wherein the medicament is formulated for the administration of 3 to 10 μg/ml of said chloroform fraction for a period of 10 minutes to the subject.

19. The use of claim 17 or 18 wherein the subject is a human or animal subject.

20. Use of a therapeutically effective amount of a chloroform fraction of a crude extract obtainable from the plant *Salvadora persica,* optionally with conventional additives, in the manufacture of a medicament for treating cholinergic conditions such as bronchial asthma in a subject.

21. The use of claim 20, wherein the medicament is formulated for administration of 3 to 10 μg/ml of said chloroform fraction for a period of 10 minutes to a subject.

22. The use of claim 20 or 21, wherein the subject is a human or animal subject.

**Patentansprüche**

1. Verfahren zur Extraktion, Fraktionierung, Reinigung und Identifizierung von biologisch wirksamen Metaboliten aus biologisch wirksamem Extrakt der Pflanze *Salvadora persica,* umfassend die Schritte des:

    a) Sammelns der Pflanzenteile von *Salvadora persica,*
    b) Lufttrocknens der Pflanzenteile;
    c) Eintauchens der Pflanzenteile in etwa 90% wäßriges Methanol für etwa eine Woche bei Raumtemperatur von etwa 28 ± 2°C,
    d) Filterns des methanolischen Extraktes,
    e) Eindampfens des methanolischen Extraktes unter vermindertem Druck bei einer Temperatur von etwa 50°C auf eine minimale Menge, um ein Rohextrakt zu erhalten,
    f) Fraktionierens des Rohextraktes unter Verwendung von Lösungsmitteln ansteigender Polarität,

g) Identifizierens der biologisch wirksamen Fraktion, und

h) Isolierens biologisch aktiver Verbindungen aus der Fraktion von Schritt (g) durch Säulenchromatographie.

2. Verfahren nach Anspruch 1, wobei die Pflanzenteile von *Salvadora persica* ausgewählt sind aus Blättern, Stielen und Blüten.

3. Verfahren nach Anspruch 1, wobei Lösungsmittel ansteigender Polarität Petrolether, Chloroform, Butanol und eine wäßrige Fraktion sind.

4. Verfahren nach Anspruch 1, wobei die Chloroformfraktion anticholinerge Wirkung, antispasmodische Wirkung und antiarrhythmische Wirkung zeigt.

5. Verfahren nach Anspruch 1, wobei die Chloroformfraktion durch eine Silicagelsäule geführt wird und dann eluiert wird unter Verwendung eines Petrolether-Ethylacetat-Chloroform-Methanol-Gradientensystems.

6. Verfahren nach Anspruch 1, wobei die Dünnschichtchromatographie (TLC) auf Silicagel ausgeführt wurde.

7. Verfahren nach Anspruch 1, wobei die TLC entweder durch Exponieren der Platten gegenüber Joddämpfen oder durch Besprühen mit methanolischer Schwefelsäure visualisiert wurde.

8. Verfahren nach Anspruch 1, wobei die Fraktionen, welche anticholinerge Wirkung zeigten, zusammengestellt wurden und durch eine Silicagelsäule geführt wurden und eluiert wurden mit demselben Satz von Lösungsmitteln.

9. Pharmazeutische Zusammensetzung, die eine anticholinerge Wirkung, antispasmodische Wirkung und antiarrhythmische Wirkung zeigt, wobei die Zusammensetzung die Chloroformfraktion von dem aus der Pflanze *Salvadora persica* erhältlichen Extrakt umfaßt, optional mit konventionellen Zusätzen.

10. Zusammensetzung nach Anspruch 9, wobei die Zusätze gewählt sind aus therapeutisch verträglichen Zusätzen.

11. Zusammensetzung nach Anspruch 9, wobei der Extrakt Verbindungen enthält, die gewählt sind aus β-Amyrin, Betulin, Ursolsäure, Methylpalmitat und Lupeol.

12. Zusammensetzung nach Anspruch 9, wobei die Extraktmenge in der Zusammensetzung 10 μg ist.

13. Zusammensetzung nach Anspruch 9, wobei die Dosierung des Extraktes 3 μg/ml bis 10 μg/ml ist.

14. Verwendung einer therapeutisch wirksamen Menge einer Chloroformfraktion, erhältlich aus der Pflanze *Salvadora persica,* optional mit konventionellen Zusätzen, bei der Herstellung eines Medikaments zur Behandlung von Arrhythmien bei einem Subjekt.

15. Verwendung nach Anspruch 14, wobei das Medikament formuliert wird zur Verabreichung von 3 bis 10 μg/ml der Chloroformfraktion für einen Zeitraum von 10 Minuten an ein Subjekt.

16. Verwendung nach Anspruch 14 oder 15, wobei das Subjekt ein menschliches oder tierisches Subjekt ist.

17. Verwendung einer therapeutisch wirksamen Menge einer Chloroformfraktion des Rohextrakts der Pflanze *Salvadora persica,* optional mit konventionellen Zusätzen, bei der Herstellung eines Medikaments zur Behandlung von krampfartigen Zuständen wie Muskelspasmen bei einem Subjekt.

18. Verwendung nach Anspruch 17, wobei das Medikament formuliert wird für die Verabreichung von 3 bis 10 μg/ml der Chloroformfraktion für einen Zeitraum von 10 Minuten an das Subjekt.

19. Verwendung nach Anspruch 17 oder 18, wobei das Subjekt ein menschliches oder tierisches Subjekt ist.

20. Verwendung einer therapeutisch wirksamen Menge einer Chloroformfraktion von einem Rohextrakt, erhältlich aus der Pflanze *Salvadora persica,* optional mit konventionellen Zusätzen, bei der Herstellung eines Medikaments zur Behandlung von cholinergen Zuständen wie Bronchialasthma bei einem Subjekt.

**21.** Verwendung nach Anspruch 20, wobei das Medikament formuliert wird zur Verabreichung von 3 bis 10 μg/ml der Chloroformfraktion für einen Zeitraum von 10 Minuten an ein Subjekt.

**22.** Verwendung nach Anspruch 20 oder 21, wobei das Subjet ein menschliches oder tierisches Subjekt ist.

## Revendications

**1.** Procédé pour l'extraction, le fractionnement, la purification et l'identification de métabolites biologiquement actifs à partir d'un extrait biologiquement actif de la plante *Salvadora persica,* comprenant les étapes de :

    a. récolte de parties de la plante *Salvadora persica,*
    b. séchage à l'air des parties de plante,
    c. immersion des parties de plante dans du méthanol aqueux à environ 90 % pendant environ une semaine à une température ambiante d'environ 28 ± 2°C,
    d. filtration de l'extrait méthanolique,
    e. évaporation de l'extrait méthanolique sous pression réduite à une température d'environ 50°C, jusqu'à une quantité minimale pour obtenir un extrait brut,
    f. fractionnement de l'extrait brut en utilisant des solvants de polarité croissante,
    g. identification de la fraction active biologiquement, et
    h. isolement des composés biologiquement actifs de la fraction de l'étape (g) par chromatographie sur colonne.

**2.** Procédé selon la revendication 1, dans lequel les parties de la plante *Salvadora persica* sont choisies parmi les feuilles, les tiges et les fleurs.

**3.** Procédé selon la revendication 1, dans lequel les solvants de polarité croissante sont l'éther de pétrole, le chloroforme, le butanol et une fraction aqueuse.

**4.** Procédé selon la revendication 1, dans lequel la fraction chloroformique présente une activité anti-cholinergique, anti-spasmodique et anti-arythmique.

**5.** Procédé selon la revendication 1, dans lequel on fait passer la fraction chloroformique dans une colonne de gel de silice, puis on l'élue en utilisant un système de gradient éther de pétrole - acétate d'éthyle - chloroforme - méthanol.

**6.** Procédé selon la revendication 1, dans lequel la chromatographie sur couche mince (CCM) a été mise en oeuvre sur du gel de silice.

**7.** Procédé selon la revendication 1, dans lequel la CCM a été révélée par exposition des plaques à des vapeurs d'iode ou par pulvérisation d'acide sulfurique méthanolique.

**8.** Procédé selon la revendication 1, dans lequel on a rassemblé les fractions qui ont présenté une activité anticholinergique, on les a fait passer dans une colonne de gel de silice puis on les a éluées avec la même série de solvants.

**9.** Composition pharmaceutique présentant une activité anti-cholinergique, anti-spasmodique et anti-arythmique, ladite composition comprenant la fraction chloroformique de l'extrait pouvant être obtenu à partir de la plante *Salvadora persica,* optionnellement avec des additifs conventionnels.

**10.** Composition selon la revendication 9, dans laquelle les additifs sont choisis parmi les additifs thérapeutiquement acceptables.

**11.** Composition selon la revendication 9, dans laquelle l'extrait contient des composés choisis parmi la β-amyrine, la bétuline, l'acide ursolique, le palmitate de méthyle et le lupéol.

**12.** Composition selon la revendication 9, dans laquelle la quantité d'extrait dans la composition est de 10 μg.

**13.** Composition selon la revendication 9, dans laquelle la dose d'extrait est de 3 μg/ml à 10 μg/ml.

**14.** Utilisation d'une quantité thérapeutiquement efficace d'une fraction chloroformique pouvant être obtenue à partir de la plante *Salvadora persica,* optionnellement avec des additifs conventionnels, dans la fabrication d'un médicament pour le traitement des arythmies chez un sujet.

**15.** Utilisation selon la revendication 14, dans laquelle le médicament est formulé pour l'administration de 3 à 10 $\mu$g/ml de ladite fraction chloroformique pendant une période de dix minutes à un sujet.

**16.** Utilisation selon la revendication 14 ou 15, dans laquelle le sujet est un sujet humain ou animal.

**17.** Utilisation d'une quantité thérapeutiquement efficace d'une fraction chloroformique de l'extrait brut de la plante *Salvadora persica,* optionnellement avec des additifs conventionnels, dans la fabrication d'un médicament pour le traitement d'états spasmodiques comme des spasmes musculaires chez un sujet.

**18.** Utilisation selon la revendication 17, dans laquelle le médicament est formulé pour l'administration de 3 à 10 $\mu$g/ml de ladite fraction chloroformique pendant une période de dix minutes au sujet.

**19.** Utilisation selon la revendication 17 ou 18, dans laquelle le sujet est un sujet humain ou animal.

**20.** Utilisation d'une quantité thérapeutiquement efficace d'une fraction chloroformique d'un extrait brut pouvant être obtenu à partir de la plante *Salvadora persica,* optionnellement avec des additifs conventionnels, dans la fabrication d'un médicament pour le traitement d'états cholinergiques comme de l'asthme bronchique chez un sujet.

**21.** Utilisation selon la revendication 20, dans laquelle le médicament est formulé pour l'administration de 3 à 10 $\mu$g/ml de ladite fraction chloroformique pendant une période de dix minutes à un sujet.

**22.** Utilisation selon la revendication 20 ou 21, dans laquelle le sujet est un sujet humain ou animal.

FIG.1(a)

FIG.1(b)

FIG.1

# CRUDE EXTRACT

FRACTIONS | Petroleum ether | Chloroform | n-Butanol | Aqueous

SUBFRACTIONS | 1-3 | 4-6 | 7-12

SUBFRACTIONS | 1-3 | 4-6 | 7-9 | 10-11

SUBFRACTIONS | 1 | 2-5

SUBFRACTIONS | 1 | 2 | 3

COMPOUNDS

β-amyrin | Methyl palmitate | Lupeol | Betulin | Ursolic acid

FIG. 2

β-AMYRIN

FIG. 3

BETULIN

FIG. 4

URSOLIC ACID

FIG. 5

$$CH_3-O-\overset{\overset{\displaystyle O}{\|}}{C}-(CH_2)_{13}-CH_3$$

METHYL PALMITATE

FIG. 6

LUPEOL

FIG. 4

Crude extract

Crude extract

```
                              |
      ┌───────────┬───────────┴──────────────┬──────────────┐
 Petroleum ether  Chloroform fraction    n-Butanol fraction  Aqueous fraction
                  Anticholinergic            Inactive            Tocolytic
                       |
        ┌──────────┬───┴────┬──────────────┐
     Fr 1-3     Fr 4-6    Fr. 7-10       Fr. 11-12
     Inactive  Anticholinergic Inactive   Inactive
                  |
        ┌─────────┼──────────┬──────────┐
     Fr i-ii   Fr. iv-vi   Fr. vii-   Fr x-xi
  Anticholinergic Inactive  Mild       Inactive
                            antispasmodic
                  |
     ┌──────┬─────┼──────┬──────┐
   Fr I   Fr II  Fr III Fr IV  Fr V
 Anticholinergic Inactive Inactive Inactive Inactive
     |
  ┌──┴───────┬──────────┐
  a          b          c
Anticholinergic Mild Anticholinergic Anticholinergic
  |            |          |
 ┌┴──┐         E        ┌─┴──┐
 A   D      Inactive    B    C
Inactive Anticholinergic Mild  Inactive
                       anticholinergic
```

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5910307 A
- US 5804206 A
- US 5607741 A
- US 5804575 A
- US 5962527 A
- US 6048847 A
- US 5948460 A

- US 5190979 A
- US 5773014 A
- US 5679393 A
- US 4808574 A
- US 6049013 A
- US 5908628 A

**Non-patent literature cited in the description**

- **NAZARINE, F ; ANITA F ; RATABOLI, P.V. ; DINIZ D'SOUZA, R. S ; DHUME; V.G.** *Indian Journal of marine Sciences,* 1998, vol. 27, 499-501
- **PISHA E ; CHAI H ; LEE IS ; CHAGWEDERA TE ; FRAMSWORTH NR ; CORDELL GA ; BEECHER CW ; FONG H H ; KINGHORN AD ; BROWN DM.** *Nature medicine,* 1995, vol. 1, 1046-1051

- **FUJIOKA T ; KASHIWADA Y ; KILKUSKIE RE ; COSENTINO LM ; BALLAS LM ; JIANG JB ; JAN-ZEN WP ; CHEN IS ; LEE KH.** *J.Nat.Prod.,* 1994, vol. 57 (2), 243-247